# EUROPÄISCHE PATENTSCHRIFT

(11) **EP 3 838 314 B1**
(45) Veröffentlichungstag und Bekanntmachung des Hinweises auf die Patenterteilung: **18.09.2024**
(21) Anmeldenummer: 20211852.7
(22) Anmeldetag: 12.05.2015
(51) Int. Cl.: A61M 11/00, A61M 11/06

(54) **VERNEBLER UND VERFAHREN ZUM HERSTELLEN EINES VERNEBLERS**
NEBULIZER AND METHOD FOR PRODUCING A NEBULIZER
NÉBULISEUR ET PROCÉDÉ DE FABRICATION D'UN NÉBULISEUR

(30) Priorität: 31.07.2014 DE 102014215064
(43) Veröffentlichungstag der Anmeldung: 23.06.2021
(62) Teilanmeldung aus: 15722188.8
(73) Patentinhaber: PARI GmbH Spezialisten für effektive Inhalation, 82319 Starnberg (DE)
(72) Erfinder: Schuschnig, Uwe, 81371 München (DE); Krüger, Ulf, 80799 München (DE); Selzer, Titus, 80686 München (DE); Lu, Erman, 28357 Bremen (DE); Jelovac, Emir, 81245 München (DE); Gramann, Jens, 82166 Gräfelfing (DE)
(74) Vertreter: Hoffmann Eitle

(56) Entgegenhaltungen:
- EP-A2- 0 786 263
- WO-A1-01/56639
- WO-A1-99/42154
- WO-A2-2013/030117
- US-A1- 2004 031 485

## Beschreibung

Die Erfindung betrifft einen Vernebler mit einem Aerosolerzeuger sowie ein Verfahren zur Herstellung eines Verneblers.

Aus der DE 19602628 A1, sind Vernebler mit einer in einem Verneblungsraum angeordneten Düse und einem in den Verneblungsraum ragenden Zuluftkamin bekannt.

Die WO 2013/030117 A2 offenbart eine Fluidkartusche zum Abgeben eines Fluids, wobei die Fluidkartusche aufweist ein Gehäuse zum Aufnehmen des Fluids, eine Druckzuführungsschnittstelle, die so konfiguriert ist, dass sie mit einer Druckzuführungseinheit zum Zuführen von Druckmedium zu dem Fluid in dem Gehäuse gekoppelt ist, und eine Fluidabgabeeinheit, die so konfiguriert ist, dass sie Partikel, insbesondere Fluidpartikel, beim Zuführen von Druckmedium zu dem Fluid in dem Gehäuse erzeugt, und einen Fluidpfad in dem Gehäuse, der geöffnet oder geöffnet werden kann, damit die Partikel, insbesondere Fluidpartikel, das Gehäuse durch den Fluidpfad verlassen können.

Des weiteren sind folgende Vernebler mit einem Aerosolerzeuger bekannt: US 2004/0031485, WO 01/56639, WO 99/42154, EP0786263 und US 2011 /0114090.

Die Erfindung ist in unabhängigen Ansprüchen 1 und 10 definiert. Die abhängigen Ansprüche sind auf bevorzugte Ausführungsformen gerichtet.

Aufgabe der Erfindung ist es, einen Vernebler bereitzustellen, mit dem eine hohe Outputrate erzielt werden kann sowie ein Verfahren zur Herstellung eines solchen Verneblers. Diese Aufgabe wird gelöst durch einen Vernebler mit den Merkmalen in Anspruch 1 bzw. ein Verfahren mit den Merkmalen des Anspruchs 10.

Unter Outputrate wird die Menge an Aerosol, die über die Zeit den Auslass passiert, verstanden.

Ein Aerosol ist ein Gemisch aus Schwebeteilchen in einem Gas. Die Schwebeteilchen können fest oder flüssig sein. Es können auch feste und flüssige Schwebeteilchen vorhanden sein. Aerosoltropfen sind Flüssigkeitstropfen in einem Gas.

Ein Vernebler ist eine Vorrichtung, die dazu eingerichtet ist, ein Aerosol bereitzustellen und abzugeben.

Ein Aerosolerzeuger ist eine Vorrichtung, die dazu eingerichtet ist, ein Aerosol zu erzeugen. Ein Aerosolerzeuger ist eine Vorrichtung, die eingerichtet ist, Flüssigkeitstropfen zu erzeugen und mit einem Gas zu vermischen. Ein Aerosolerzeuger weist bevorzugt einen Vernebler, einen Zerstäuber, einen Befeuchter, einen Druckluftvernebler, einen Luftzerstäuber, einen elektronischen Vernebler, einen Ultraschallvernebler, einen elektrohydrodynamischen Vernebler, einen elektrostatischen Vernebler, einen Membranvernebler, einen Vernebler mit einer vibrierenden Membran, einen elektronischen Vernebler mit einer vibrierenden Membran, einen Mesh-Vernebler, einen Düsenvernebler, einen Inhalator (MDI), einen Pulverzerstäuber (DPI) oder eine Kombination davon auf. Der Inhalator weist in einer Ausführungsform einen unter Druck stehenden Kanister mit einem Medikament und einem Treibgas auf. Zweckmäßigerweise ist der Kanister mit einem von Hand bedienbaren Aktuator verbunden. Es ist vorteilhaft, wenn der Inhalator eingerichtet ist, bei der Aktivierung eine bestimmte Medikamentenmenge in Aerosolform abzugeben. In einer Ausführungsform ist die Aerosolerzeugungsvorrichtung für den Einsatz mit Beatmungsvorrichtungen eingerichtet.

Ein Verneblungsraum ist ein Raum, der dazu eingerichtet ist, von Aerosol durchströmt zu werden. Ein Verneblungsraum kann ein Raum sein, der dazu eingerichtet ist, ein Aerosol zumindest zeitweise aufzunehmen.

Ein Auslass ist eine Einrichtung, die dazu vorgesehen ist, ein Aerosol aus dem Verneblungsraum herauszulassen. Zweckmäßigerweise ist der Auslass dazu eingerichtet, mit einem Bauteil, wie einem Mundstück oder einer Maske verbunden zu werden.

Ein Kunststoffbauteil ist ein Bauteil, das einen Kunststoff aufweist. Das Bauteil kann vollständig aus Kunststoff bestehen. Der Kunststoff kann ein organischer, polymerer Festkörper, der synthetisch oder halbsynthetisch aus monomeren organischen Molekülen oder Biopolymeren hergestellt worden ist, sein. Der Kunststoff kann ein Thermoplast, Duroplast oder Elastomer sein. Das Kunststoffbauteil kann beispielsweise ein Polypropylen aufweisen.

Eine Benetzungsoberfläche ist eine Oberfläche, die dazu eingerichtet ist, von einer Flüssigkeit benetzt zu werden. Eine Benetzung findet statt, wenn eine Flüssigkeit mit der Benetzungsoberfläche einen Kontaktwinkel von 90 Grad oder weniger bildet.

In einer Ausführungsform weist die Benetzungsoberfläche zumindest vorübergehend, vorzugsweise dauerhaft, eine Oberflächenspannung von 32 bis 5000 Millinewton pro Meter (mN/m) auf. Vorzugsweise liegt die Oberflächenspannung zwischen 35 und 400 Millinewton pro Meter (mN/m), besonders bevorzugt zwischen 44 und 73 Millinewton pro Meter (mN/m). Die Oberflächenspannung kann zwischen 50 und 72 Millinewton pro Meter (mN/m) liegen.

Die Benetzungsoberfläche kann ein Benetzungsmaterial aufweisen. Bevorzugte Benetzungsmaterialien sind Metalle und Glas. In einer Ausführungsform weist die Benetzungsoberfläche ABS auf.

Die Benetzungsoberfläche kann eine vorgegebene Oberflächenrauheit aufweisen. Der Mittenrauwert Rₐ kann zwischen 6,3 und 0,006 liegen. Bevorzugt liegt der Mittenrauwert Rₐ zwischen 1,6 und 0,01, besonders bevorzugt zwischen 0,5 und 0,006. Vorzugsweise wird die Oberflächenrauheit durch eine entsprechend eingerichtete Gussform, wie eine Spritzgussform oder Druckgussform und einen entsprechenden Fertigungsprozess bereitgestellt.

Die Benetzungsoberfläche kann gereinigt sein.

Die Benetzungsoberfläche kann eine Temperatur zwischen 20 und 80 Grad, vorzugsweise zwischen 40 und 50 Grad aufweisen.

Die Benetzungsoberfläche kann eine Oberflächenbehandlung aufweisen.

Vorzugsweise ist die Benetzungsoberfläche bedruckt oder beschichtet, besonders bevorzugt ist sie lackiert oder beklebt.

Die Benetzungsoberfläche kann gestrahlt sein. Sie kann insbesondere mit Sand, Glasperlen, Keramik, Stahl, Trockeneis, Korund, Stahlkies, Stahlkugeln, Drahtkorn, Schmirgel, Bronzekies, Hochofenschlacke, Kalziumkarbonat-Granulat oder Kunststoffen gestrahlt sein.

Die Benetzungsoberfläche kann korona-behandelt, flammbehandelt, fluoriert, plasmabehandelt, insbesondere sauerstoff-plasmabehandelt oder niederdruckplasmabehandelt, ozonbehandelt oder UV-Licht behandelt sein. Besonders bevorzugt ist die Benetzungsoberfläche eine fluorierte Benetzungsoberfläche. Die fluorierte Benetzungsoberfläche ist vorzugsweise durch Ersetzen von Wasserstoffatomen einer Polymerkette durch Fluoratome erzielt worden. Die fluorierte Oberfläche oder fluorierte Benetzungsoberfläche ist Fluor, zweckmäßigerweise einem Fluorgemisch ausgesetzt worden. Vorzugsweise ist dies bei Raumtemperatur, besonders bevorzugt bei 25° C, erfolgt. Es ist zweckmäßig, wenn dabei keine Temperaturspitzen aufgetreten sind. Die Eindringtiefe der Fluoratome in das Substrat liegt vorzugsweise im molekularen Bereich. Es ist bevorzugt, wenn die Eigenschaften des Basismaterials unverändert geblieben sind. Das Basismaterial weist Kunststoff auf, vorzugsweise weist das Basismaterial ABS, EPDM, Kunststoff aus nachwachsenden Rohstoffen, NBR, HNBR, PEEK, PMMA, Polyamid, Polyester, Polyethylen, LDPE, PE-UHMW, POM, PET, EPDM, PBT, Polycarbonat, Polyphenylsulfid, Polysiloxan-Elastomer, glasfaserhaltiges Polymer, Mischpolymer, Polypropylen, Silikon, Kautschuk oder TPE auf. Die Oberflächenenergie der Benetzungsoberfläche kann der Oberflächenenergie einer Flüssigkeit angepasst worden sein. Die Flüssigkeit weist bevorzugt Kochsalzlösung oder ein Medikament auf.

In einer Ausführungsform ist die fluorierte Benetzungsoberfläche durch Gasphasenfluorierung erzielt worden. Bei der Gasphasenfluorierung wird das Objekt oder Bauteil Fluor, zweckmäßigerweise einem Fluorgemisch in Gasform ausgesetzt, so dass in der äußersten Randschicht Wasserstoffatome durch Fluoratome ersetzt werden. Dadurch kann eine Oberfläche eines Bauteils vergleichsweise geometrieunabhängig behandelt werden. Vorzugsweise ist die fluorierte Benetzungsoberfläche durch Behandlung des Bauteils in einem Vakuumreaktor erzielt worden. Das fluorierte Bauteil kann in einer vorzugsweise zylindrischen Kammer als Schüttgut behandelt worden sein. Es ist vorteilhaft, wenn das fluorierte Bauteil in eine vorzugsweise kubische Kammer in einem Korb, in einer Gitterbox oder in einem speziellen Ladungsträger eingebracht worden ist. Das fluorierte Bauteil kann in einem Durchlaufreaktor behandelt worden sein.

In einer Ausführungsform ist die fluorierte Benetzungsoberfläche erzielt worden, indem fluoriertes Pulver oder Granulat durch Spritzgießen oder Blasformen weiterverarbeitet wurde. Dabei kann das Pulver oder Granulat derart fluoriert worden sein, dass die Fluoratome sich nur in einem äußeren Bereich der Pulverteilchen oder der Granulatkörnchen befinden. Die Fluoratome können auch tiefer in die Pulverteilchen oder Granulatkörnchen eingedrungen sein oder sich in allen Bereichen der Pulverteilchen oder Granulatkörnchen befinden. Das Pulver oder Granulat ist zweckmäßigerweise durch Gasphasenfluorierung fluoriert worden. Dazu ist vorzugsweise Polyolefin-Pulver oder -Granulat eingesetzt worden. Die Fluorierung des Pulvers oder Granulats ist bevorzugt in einer Drehtrommel mit Schaufeleinbauten oder in einem Reaktor mit beweglichem Rührer erzeugt worden. Es ist zweckmäßig, wenn der Reaktor zylindrisch ist und der Rührer eingerichtet ist, zu rotieren.

Dadurch kann eine über längere Zeit aktive Oberfläche mit stark polarem Charakter erzielt worden sein, ohne das Basismaterial zu beeinflussen. Die Fluorierung der Oberfläche ist bevorzugt nicht reversibel. Es kann eine Sperrschicht, bevorzugt eine Permeationsschicht aufgebaut worden sein. Permeation, Diffusion, Migration, Reibung, Anhaftung und Klebrigkeit können reduziert worden sein. Haftung, Filmbildung, Adhäsion und Benetzbarkeit können erhöht worden sein. Es kann ein besseres Ablaufen von Flüssigkeiten, ein schnelleres Trocknen, eine höhere Druckqualität, bessere Sauberkeit, da Schmutzpartikel nicht anhaften, sowie geringere Kalk- und Schimmelbildung, bessere Montierbarkeit, oder eine optisch ansprechendere Oberfläche erreicht worden sein. Durch die Sperrschicht kann verhindert werden, dass Bestandteile von Kunststoffmaterial aus der Oberfläche diffundieren oder eine Behälterfüllung eine Wandung durchdringt. Geruchsbelästigung, Umweltbelastung, Aufquellen, eine schmierige Oberfläche und Etikettenablösung können verhindert werden. Die erreichte Eigenschaft kann auch nach Reinigungs- und Sterilisationsvorgängen langzeitstabil sein. Eine Oberfläche eines Werkstücks kann derart fluoriert sein, dass die Maße des Werkstücks vor und nach dem Fluorieren gleich sind. Es kann eine antibakterielle oder antimikrobielle Wirkung erzielt worden sein. Die antibakterielle oder antimikrobielle Wirkung kann vorübergehend sein.

Die Benetzungsoberfläche kann einer Oberflächenaktivierung unterzogen worden sein.

Durch das Vorsehen der Benetzungsoberfläche wird erreicht, dass Flüssigkeitstropfen, die an eine Oberfläche des Verneblungsraums gelangen, sich an die Benetzungsoberfläche anlegen. Es wird ein Flüssigkeitsfilm gebildet. Es kann auch der Effekt erzielt werden, dass Flüssigkeit von der Oberfläche abgleitet.

Dies ist vorteilhaft, da sich an Oberflächen, die nicht oder nur schlecht benetzbar sind, Flüssigkeitstropfen bilden können, die weit von der Oberfläche abstehen. Dadurch kann ein durchströmbarer Querschnitt des Verneblungsraums verringert werden. Ein verringerter Querschnitt kann bewirken, dass vermehrt Aerosoltropfen an den Oberflächen des Verneblungsraums und den bereits an der Oberfläche vorhandenen Flüssigkeitstropfen auftreffen und anhaften. Diese Aerosoltropfen können dann nicht zum Auslass gelangen.

Außerdem können sich von einer schlecht benetzbaren Oberfläche große Tropfen lösen und durch einen von einem Aerosol durchströmten Raum herabtropfen. Dabei werden Aerosoltropfen mitgenommen, die dann nicht mehr zum Auslass gelangen können. Dadurch wird die Outputrate gesenkt.

In einer Ausführungsform ist eine Benetzungsoberfläche derart vorgesehen, dass sich darauf ein Flüssigkeitsfilm bilden kann und dass Tropfen, die auf den Flüssigkeitsfilm prallen, weitere Tropfen erzeugen können.

In einer Ausgestaltung, lässt sich die respirable Rate um bis zu 30 % erhöhen. Vorzugsweise ist dazu eine Oberflächenbehandlung an einem Düsenaufsatz und einem Verneblerunterteil vorgesehen. In bevorzugten Ausgestaltungen kann die Therapiedauer um 20 % reduziert werden, kann das Residualvolumen reduziert werden, kann der Patient in kürzerer Zeit eine höhere Dosis Medikament erhalten und kann der Verneblerwirkungsgrad erhöht werden. Bevorzugt bleiben diese Eigenschaften auch nach Reinigung in der Geschirrspülmaschine und nach 10 Autoklavierzyklen bei 134°C erhalten.

In einer Ausführungsform weist der Aerosolerzeuger eine Düse auf. In der Kombination mit einer Düse ist es besonders sinnvoll, eine Benetzungsoberfläche vorzusehen, da Düsen Aerosole erzeugen können, deren Partikel große Durchmesserunterschiede aufweisen. Dadurch haften eher sehr große Flüssigkeitstropfen an Oberflächen des Verneblungsraums an. Sie können einen zu durchströmenden Querschnitt stark verkleinern und die Outputrate stark senken.

Vorzugsweise ist die Düse mit einer Flüssigkeitszufuhr versehen und dazu eingerichtet, von Gas durchströmt zu werden. Die Düse kann als Zweistoffdüse innerer Mischung oder als Zweistoffdüse äußerer Mischung ausgeführt sein.

Es ist zweckmäßig, dass der Verneblungsraum eine Aerosolführung aufweist, die den Aerosolerzeuger mit dem Auslass verbindet, und dass die Aerosolführung die Benetzungsoberfläche aufweist. Ein großer Teil des Aerosols, das geeignet ist, zum Auslass geführt zu werden, kann so zum Auslass gelangen.

Die Aerosolführung ist vorzugsweise ein Hohlraum, durch den sich das erzeugte Aerosol hindurchbewegen muss, um zu dem Auslass zu gelangen. Zweckmäßigerweise weist die Aerosolführung einen durchgehenden Pfad auf, an dem entlang das Aerosol durch die Aerosolführung gelangen kann.

Die Aerosolführung kann teilweise mit der Benetzungsoberfläche versehen sein, vorzugsweise ist sie vollständig mit der Benetzungsoberfläche ausgekleidet.

In einer Ausführungsform weist eine Fläche der Aerosolführung die Benetzungsoberfläche auf. Da sich Flächen leicht bearbeiten lassen, kann so auf einfache Weise ein Querschnitt der Aerosolführung zumindest weitgehend aufrechterhalten werden.

Eine Fläche ist vorzugsweise eine zweidimensionale Begrenzungsfläche eines Körpers. Eine Fläche kann eben sein, eine Fläche kann auch eine Krümmung aufweisen.

Wenn eine Kante der Aerosolführung die Benetzungsoberfläche aufweist, kann erreicht werden, dass sich Flüssigkeit besonders gut an die Kante anlegt. Bevorzugt ist die Benetzungsoberfläche derart ausgestaltet, dass Flüssigkeit gut von der Kante abfließt. Der Effekt, dass Flüssigkeit sich derart an der Kante anlagert, dass die Kante verlegt wird, kann verringert oder vermieden werden.

Dies ist besonders bei Kanten wünschenswert, die eine Funktion haben. Die Funktion kann das Bereitstellen eines definierten Querschnitts, wie bei einer Blende, sein. Eine Funktion kann auch das Vorgeben eines definierten Pfades zur Ausfilterung von unerwünschten Bestandteilen im Aerosol sein. Die Kante kann dabei eine Filterfläche, wie unten beschrieben, begrenzen, die dazu vorgesehen ist, Aerosoltropfen mit einem vorgegebenen Durchmesser aus einem Aerosolstrom herauszufiltern.

In einer Ausführungsform weist die Aerosolführung einen Durchlass auf und der Durchlass weist die Benetzungsoberfläche auf. Dadurch kann erreicht werden, dass der Querschnitt der Aerosolführung an einer für die Outputrate besonders wichtigen Stelle weitgehend oder vollständig erhalten bleibt.

Ein Durchlass ist vorzugsweise ein Bereich in der Aerosolführung, der einen kleineren Querschnitt aufweist als daran angrenzende Bereiche. Ein Durchlass kann eine Verengung sein, die eingerichtet ist, um Drücke oder Geschwindigkeiten von hindurchfließenden Fluiden zu beeinflussen, wie eine Düse oder eine Blende.

Es ist vorteilhaft, wenn der Verneblungsraum eine Begrenzungswand aufweist, die die Benetzungsoberfläche aufweist. Auf diese Weise kann erreicht werden, dass vergleichsweise große Flüssigkeitsmengen zügig in ein Reservoir zurückgeführt werden. Außerdem kann so die Restmenge, die nach Gebrauch in dem Vernebler verbleibt und dem Patienten nicht zur Verfügung gestellt wird, besonders gut reduziert werden.

Eine Begrenzungswand ist eine Wand, die den Verneblungsraum zur Umgebung abgrenzt.

Der Verneblungsraum kann eine Luftzuführung aufweisen, die die Benetzungsoberfläche aufweist. Die zugeführte Luft kann so besonders präzise geleitet werden.

Eine Luftzuführung ist derart eingerichtet, dass sie es Umgebungsluft ermöglichen kann, in den Verneblungsraum zu gelangen. In einer Ausführungsform ist die Luftzuführung eingerichtet, Umgebungsluft an dem Aerosolerzeuger vorbei zu dem Auslass zu leiten. Dadurch kann das Aerosol effektiv zu dem Auslass geführt werden.

Bevorzugt ist die Luftzuführung als Zuluftkamin ausgebildet.

In einer Ausführungsform weist der Verneblungsraum eine Prallfläche auf, und die Prallfläche weist die Benetzungsoberfläche auf. So kann erreicht werden, dass Aerosoltropfen, die auf die Prallfläche aufgebracht werden, die Oberfläche und Kontur der Prallfläche möglichst wenig beeinflussen. Es kann eine gleichbleibende Funktion der Prallfläche erzielt werden.

Eine Prallfläche ist eine Fläche, die dazu eingerichtet ist, dass Aerosol sich dagegen bewegt. Bei Berührung der Prallfläche können Aerosoltropfen in kleinere Aerosoltropfen zerfallen.

Eine Prallfläche kann an einem Luftstromsteuer vorgesehen sein. Das Luftstromsteuer kann am Austritt eines Fluidstroms vorgesehen sein. In einer Ausführungsform ist das Luftstromsteuer im Fluidstrom gegenüber einer Austrittsöffnung angeordnet. Die Austrittsöffnung ist zweckmäßigerweise die Austrittsöffnung einer Düse, vorzugsweise einer Gasdüse.

Eine Prallfläche kann an einem Prallschirm vorgesehen sein. Ein Prallschirm ist ein Bauteil, das benachbart zu einem Aerosolerzeuger angeordnet ist.

Der Prallschirm ist vorzugsweise derart angeordnet, dass Aerosoltropfen, deren Durchmesser größer als ein vorgegebener Durchmesser ist, darauf prallen können. Dazu erstreckt er sich in einer Ausführungsform zumindest in einer Ebene um den Aerosolerzeuger herum. Er kann insbesondere zylindrisch, kegelstumpfförmig oder kegelförmig ausgebildet sein.

Das Vorsehen einer Benetzungsoberfläche an einem Prallschirm kann besonders vorteilhaft sein, wenn die Aerosolführung derart ausgestaltet ist, dass an dem Prallschirm ein besonders kleiner durchströmbarer Querschnitt vorhanden ist. Anhaftende Flüssigkeit könnte diesen Querschnitt noch weiter verkleinern, so dass weniger Aerosol zu dem Auslass strömen kann. Durch das Vorsehen einer Benetzungsoberfläche an dem Prallschirm kann erreicht werden, dass die an den Prallschirm gelangte Flüssigkeit sich stärker an den Prallschirm anlegen kann und sich schneller von dem Prallschirm entfernen kann.

Der Verneblungsraum weist eine Filterfläche auf, und die Filterfläche weist die Benetzungsoberfläche auf.

Um die Funktion eines Filters erfüllen zu können, ist eine Filterfläche derart angeordnet, dass vorwiegend größere Flüssigkeitstropfen dagegen prallen und kleinere Flüssigkeitstropfen daran vorbei geleitet werden. Dies wird durch die Anordnung der Filterfläche in einem Bereich, der dafür vorgesehen ist, dass ein Aerosol eine Richtungsänderung erfährt, erreicht. In derartigen Bereichen verteilen sich größere und kleinere Flüssigkeitstropfen typischerweise vorwiegend in bestimmten Teilbereichen, so dass durch die Anordnung und Ausgestaltung der Prallfläche bestimmt werden kann, welche Flüssigkeitstropfen ausgefiltert werden.

Die Kante der Filterfläche kann dabei bestimmen, welche Aerosoltropfen gerade noch ausgefiltert werden und welche Aerosoltropfen die Filterfläche passieren können.

Durch diese Funktion der Filterfläche wird sie besonders stark mit Flüssigkeitstropfen versehen. Diese Funktion erfordert aber auch eine präzise Form der Filterfläche. Die Form der Filterfläche kann durch Flüssigkeitstropfen, die sich darauf befinden, so verändert werden, dass sich das Tropfenspektrum der ausgefilterten Flüssigkeitstropfen verändert.

Das Tropfenspektrum kennzeichnet das Vorkommen von Flüssigkeitsstropfen mit bestimmten Durchmessern im Aerosol.

Um die Veränderung der Form der Filterfläche möglichst gering zu halten, ist es sinnvoll, die Filterfläche mit einer Benetzungsoberfläche zu versehen. Dadurch können sich aufgebrachte Flüssigkeitstropfen so an die Filterfläche anlegen, dass sie die Form der Filterfläche wenig verändern. Außerdem kann die Benetzungsoberfläche derart ausgestaltet werden, dass Flüssigkeitstropfen so gut abfließen, dass sie schnell von der Filterfläche entfernt werden und sich wenig Flüssigkeit auf der Filterfläche befindet.

Eine Filterfläche kann an einem Verneblungsraumteiler vorgesehen sein. Ein Verneblungsraumteiler ist vorzugsweise dazu eingerichtet, den Verneblungsraum derart zu unterteilen dass das Aerosol eine Richtungsänderung vornehmen muss, um zu dem Auslass gelangen zu können. In Verbindung mit dieser Funktion kann der Verneblungsraumteiler nach dem oben beschriebenen Prinzip Aerosol mit erwünschtem Tropfenspektrum von Aerosol mit unerwünschtem Tropfenspektrum trennen.

Die Filterfläche kann an einer Labyrinthführung vorgesehen sein. Eine Labyrinthführung ist eine Aerosolführung, bei deren Durchströmung mindestens eine, vorzugsweise mindestens zwei oder drei Richtungsänderungen des Aerosols erforderlich sind. In Verbindung mit dieser Eigenschaft kann die Labyrinthführung nach dem oben beschriebenen Prinzip Aerosol mit erwünschtem Tropfenspektrum von Aerosol mit unerwünschtem Tropfenspektrum trennen.

Die Filterfläche kann an einem Prallschirm vorgesehen sein. Der Prallschirm ist, wie oben beschrieben, vorzugsweise derart angeordnet, dass ein vorgegebenes Tropfenspektrum dagegen geleitet wird. Dazu erstreckt er sich wie oben beschrieben in einer Ausführungsform zumindest in einer Ebene um den Aerosolerzeuger herum. Er kann insbesondere zylindrisch, kegelstumpfförmig oder kegelförmig ausgebildet sein.

Zweckmäßigerweise muss das Aerosol, um zu dem Auslass gelangen zu können, im Bereich des Prallschirms vorzugsweise benachbart zu einer Oberfläche insbesondere benachbart zu einer Kante des Prallschirms die Richtung ändern.

Dabei ist der Prallschirm zweckmäßigerweise derart angeordnet, dass er nach dem oben beschriebenen Prinzip Aerosoltropfen, die nicht zu dem Auslass gelangen sollen, daran hindert, den Weg zu dem Auslass zurückzulegen. Derartige Aerosoltropfen können auf eine Oberfläche des Prallschirms geleitet werden, so dass sie dagegen prallen und daran haften können. In einer Ausführungsform ist der Vernebler derart eingerichtet, dass Einatemluft innen durch den Prallschirm an dem Aerosolerzeuger vorbeiströmen kann, dabei Aerosol mitnimmt, um die Kante des Prallschirms herum und an einer Außenseite des Prallschirms entlang in Gegenrichtung weiterströmt. Dabei werden Aerosoltropfen, die zu groß sind, um diesem Pfad zu folgen, auf der Innenseite des Prallschirms abgelagert. Die Positionierung der Kante des Prallschirms bestimmt, welche Aerosoltropfen gerade noch auf dem Prallschirm abgelagert werden und welche Aerosoltropfen gerade noch weiter in Richtung des Auslasses gelangen können. Daher ist es auch hier für das Tropfenspektrum des zu dem Auslass geleiteten Aerosols wichtig, dass die Außenabmessungen der Kante nicht durch anhaftende Flüssigkeit verändert werden, da sich dann das ausgefilterte Tropfenspektrum ändern kann. Dadurch ist das Vorsehen einer Benetzungsoberfläche an der Kante des Prallschirms, an der Innenseite des Prallschirms oder an dem gesamten Prallschirm besonders sinnvoll.

Der Verneblungsraum weist ein Reservoir auf, und es ist zweckmäßig, wenn das Reservoir die Benetzungsoberfläche aufweist. Dadurch kann sich Flüssigkeit besonders gut in einem dafür vorgesehenen Bereich des Reservoirs ansammeln.

Ein Reservoir ist ein Speicher, der für die Bevorratung von Flüssigkeiten insbesondere für die Bevorratung von Medikamenten vorgesehen ist. Das Reservoir ist dazu eingerichtet, Flüssigkeit an den Aerosolerzeuger abzugeben. Es ist vorteilhaft, wenn der Vernebler derart eingerichtet ist, dass Flüssigkeit, die sich im Verneblungsraum auf Oberflächen abgelagert hat, in das Reservoir gelangen kann.

Im Folgenden wird die Erfindung anhand von Ausführungsbeispielen unter Bezugnahme auf die beigefügten Figuren genauer beschrieben.
Figur 1 zeigt ein Ausführungsbeispiel eines Verneblers mit einer Benetzungsoberfläche an einem Zuluftkamin,
Figur 2 zeigt einen Querschnitt eines Verneblers ohne Benetzungsoberfläche,
Figur 3 zeigt einen Querschnitt des in Figur 1 gezeigten Verneblers,
Figur 4 zeigt einen Vernebler, bei dem der Verneblungsraum vollständig mit der Benetzungsoberfläche versehen ist.
Figur 5 zeigt ein Ausführungsbeispiel eines Verneblers mit einer Benetzungsoberfläche an einem Prallschirm.
Fig. 6 zeigt einen Vernebler, mit einem Verneblungsraum, einem Mundstück und einem Membran-Aerosolgenerator.

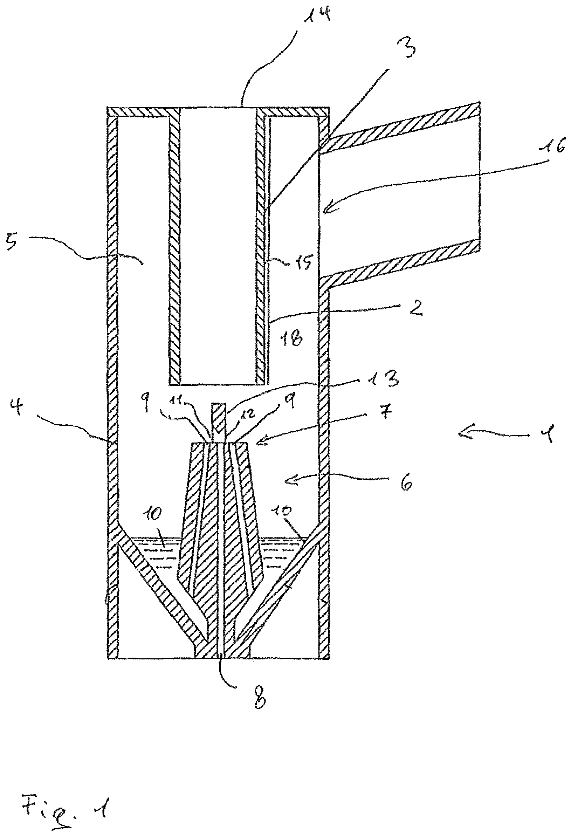

Figur 1 zeigt ein Ausführungsbeispiel eines Verneblers 1 mit einer Benetzungsoberfläche 2 an einem Zuluftkamin 3. Der in der Figur 1 gezeigte Vernebler 1 weist eine Begrenzungswand 4 auf, die einen Verneblungsraum 5 umschließt. In dem Verneblungsraum 5 befindet sich ein Aerosolerzeuger 6, der als Zweistoffdüse 7 ausgebildet ist. Die Zweistoffdüse 7 weist einen Gaskanal 8 und drei Flüssigkeitskanäle 9 auf, wobei in Figur 1 nur zwei der drei Flüssigkeitskanäle 9 gezeigt sind. Der Gaskanal 8 hat einen nicht gezeigten Anschluss für eine Druckluftquelle. Die Flüssigkeitskanäle 9 sind dazu eingerichtet, mit einem Medikamentenreservoir 10 zusammenzuwirken. Der Gaskanal 8 und die Flüssigkeitskanäle 9 münden benachbart zueinander in einer Zerstäubungsregion 11 der Zweistoffdüse 7. Gegenüber einer Mündung 12 des Gaskanals 8 ist ein Luftstromsteuer 13 vorgesehen.

Ein Zuluftkamin 3 erstreckt sich von einer Einlassöffnung 14 der Begrenzungswand 4 in Richtung der Zweistoffdüse 7. Die Außenseite 15 des Zuluftkamins 3 ist mit einer Benetzungsoberfläche 2 versehen. Die Benetzungsoberfläche 2 erstreckt sich über die gesamte Länge und die Hälfte des Umfangs des Zuluftkamins 3. Dabei erstreckt sich die Benetzungsoberfläche 2 über die Hälfte des Umfangs des Zuluftkamins 3, die einem in der Begrenzungswand 4 vorgesehenen Auslass 16 zugewandt ist.

Im Betrieb des Verneblers 1 gelangt ein flüssiges Medikament aus dem Medikamentenreservoir 10 durch die Flüssigkeitskanäle 9 zu der Zerstäubungsregion 11. Druckluft gelangt durch den Gaskanal 8 ebenfalls zu der Zerstäubungsregion 11. Beim Einatmen saugt ein Patient Umgebungsluft durch den Zuluftkamin 3 zu der Zerstäubungsregion 11. In der Zerstäubungsregion 11 entsteht mithilfe des Luftstromsteuers 13 ein Aerosol. Der Patient saugt Aerosol beim Einatmen durch den Auslass 16 aus dem Vernebler 1 heraus. Dadurch gelangt Medikamentenflüssigkeit an die Innenseite der Begrenzungswand 4 und an den Zuluftkamin 3. Diese Medikamentenflüssigkeit verkleinert den Durchlass 18 zwischen der Begrenzungswand 4 und dem Zuluftkamin 3. Dadurch kann weniger Aerosol zu dem Patienten gelangen.

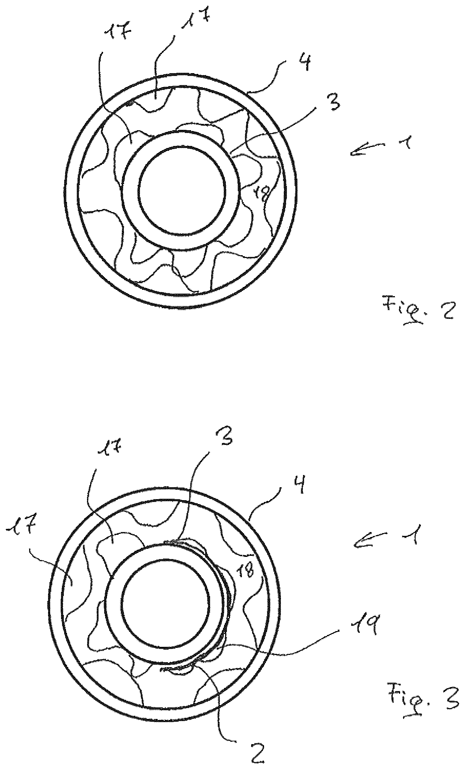

An der Benetzungsoberfläche 2 ist dieser Effekt geringer, wie im Folgenden anhand der Figuren 2 und 3 beschrieben wird.

Figur 2 zeigt einen Querschnitt eines Verneblers 1 ohne Benetzungsoberfläche. An der Begrenzungswand 4 und dem Zuluftkamin 3 befinden sich dicke Flüssigkeitstropfen 17. Der Durchlass 18 zwischen der Begrenzungswand 4 und dem Zuluftkamin 3 ist durch die dicken Flüssigkeitstropfen 17 stark verkleinert. Viele Aerosoltropfen, die von der Zerstäubungsregion 11 durch diesen Durchlass 18 in Richtung des Auslasses 16 geleitet werden, prallen auf die dicken Flüssigkeitstropfen 17 und können nicht zu dem Auslass 16 gelangen.

Figur 3 zeigt einen Querschnitt des in Figur 1 gezeigten Verneblers 1. Bei diesem Vernebler 1 befinden sich ebenfalls an der Begrenzungswand 4 und dem Zuluftkamin 3 dicke Flüssigkeitstropfen 17. Im Bereich der Benetzungsoberfläche 2 befinden sich jedoch keine dicken Flüssigkeitstropfen 17, sondern flache Flüssigkeitstropfen 19. Dies wird durch die gute Benetzbarkeit der Benetzungsoberfläche 2 erreicht. Dadurch, dass sich an der Benetzungsoberfläche 2 keine dicken Flüssigkeitstropfen 17 befinden, ist der Querschnitt des Durchlasses 18 in diesem Bereich größer. Es prallen weniger Aerosoltropfen auf die flachen Flüssigkeitstropfen 19. Dadurch ist die Outputrate größer als bei einem Vernebler 1 ohne Benetzungsoberfläche 2.

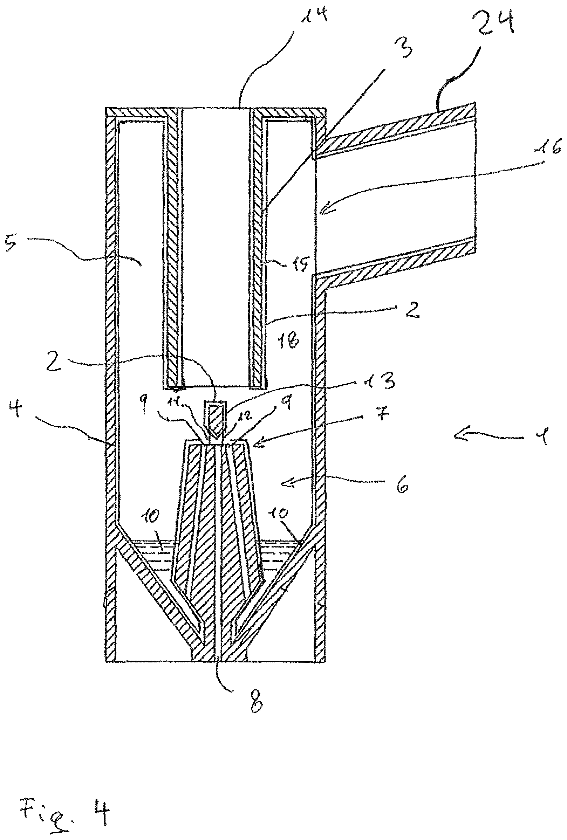

Figur 4 zeigt einen Vernebler 1, bei dem der Verneblungsraum 5 vollständig mit der Benetzungsoberfläche 2 versehen ist. Die Innenwand der Begrenzungswand 4, der Zuluftkamin 3, das Innere eines Anschlusses 24, die Zweistoffdüse 7 und das Luftstromsteuer 13 sind mit der Benetzungsoberfläche 2 versehen.

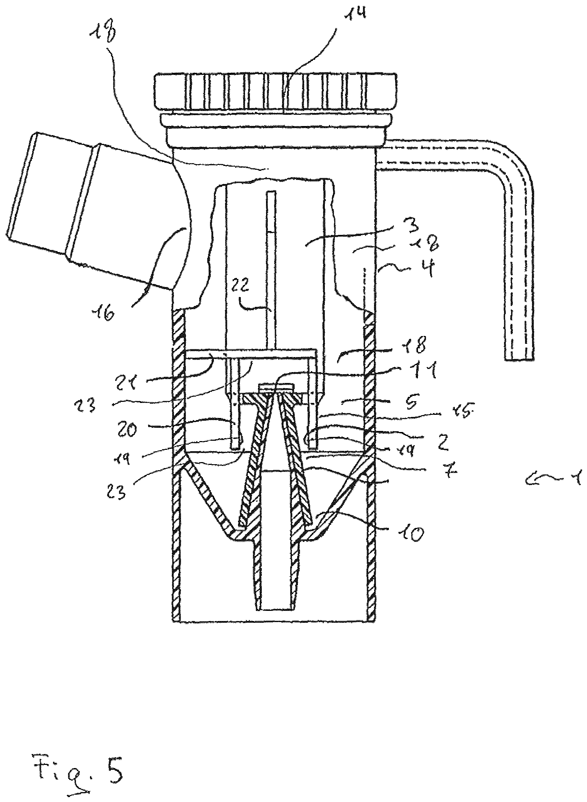

Figur 5 zeigt ein Ausführungsbeispiel eines Verneblers 1 mit einer Benetzungsoberfläche 2 an einem Prallschirm 20. Der in Figur 5 gezeigte Vernebler 1 entspricht im Wesentlichen dem in Figur 1 gezeigten Vernebler 1. Er ist jedoch zusätzlich mit einem Prallschirm 20, einem Prallabschnitt 21 und einem Leitabschnitt 22 ausgestattet.

Der Prallschirm 20 ist im Bereich der Zerstäubungsregion 11 angeordnet. Er hat im Wesentlichen die Form eines Hohlzylinders und ist derart angeordnet, dass sich die Zerstäubungsregion 11 etwa in der Mitte der Längsachse des Prallschirms 20 befindet. Dadurch erstreckt sich der Prallschirm 20 um die Zerstäubungsregion 11 herum. Der Prallschirm 20 ist an der Stirnseite 23, die in Richtung des Medikamentenreservoirs 10 weist, offen. Die Stirnseite 23 des Prallschirms 20, die näher an dem Auslass 16 angeordnet ist, ist von dem Prallabschnitt 21 abgedeckt.

Der Prallabschnitt 21 erstreckt sich in einem zum Auslass 16 benachbarten Bereich bis zur Begrenzungswand 4. Zwischen dem gegenüberliegenden Bereich der Begrenzungswand 4 und dem Prallschirm 20 ist ein Durchlass 18 vorhanden.

Der Leitabschnitt 22 erstreckt sich von dem Prallabschnitt 21 in Richtung der Einlassöffnung 14. Dabei bleibt zwischen der Begrenzungswand 4 mit der Einlassöffnung 14 und dem Leitabschnitt 22 ein Durchlass 18 bestehen. Der Leitabschnitt 22 erstreckt sich quer zwischen dem Auslass 16 und dem diesem gegenüberliegenden Bereich der Begrenzungswand 4.

Beim Betrieb des in Figur 5 gezeigten Verneblers 1 entsteht ein Aerosol nach dem gleichen Prinzip wie beim Betrieb des in Figur 1 gezeigten Verneblers 1.

Der Patient saugt ebenfalls beim Einatmen Aerosol durch den Auslass 16 aus dem Vernebler 1 heraus. Dazu muss das Aerosol sich von der Zerstäubungsregion 11 zu der offenen Stirnseite 23 des Prallschirms 20 bewegen. Dann muss das Aerosol die Richtung ändern und zwischen einer Außenseite 15 des Prallschirms 20 und der Begrenzungswand 4 durch den Durchlass 18 hindurchtreten. Von dem Durchlass 18 aus muss es in Richtung der Einlassöffnung14 und dann um den Leitabschnitt 22 herum zu dem Auslass 16 strömen.

Dieser vorgegebene Weg dient dazu, unerwünschte Aerosoltropfen auszufiltern. Aerosoltropfen, die zu groß sind, um dem vorgegebenen Aerosolstrom zu folgen, prallen auf den Prallschirm 20 oder den Leitabschnitt 22 auf. Zwischen dem Prallschirm 20 und der Zweistoffdüse 7 ist nur ein kleiner Durchtrittsbereich für das Aerosol vorhanden. Dieser wird je nach dem Flüssigkeitsstand im Medikamentenreservoir 10 durch das Medikament noch weiter eingeschränkt. Die genaue Geometrie des Prallschirms 20 bestimmt das ausgefilterte Tropfenspektrum. Durch Flüssigkeit, die sich an dem Prallschirm 20 befindet, wird sowohl der Durchtrittsbereich für das Aerosol verkleinert als auch die Geometrie verändert. Dadurch werden vermehrt Tropfen ausgefiltert, so dass die Outputrate niedrig ist. Außerdem wird das ausgefilterte Tropfenspektrum verändert.

Um diese Effekte zu minimieren, ist der Prallschirm 20 vollständig mit einer Benetzungsoberfläche 2 versehen. Nach dem in Figur 3 beschriebenen Prinzip wird durch das Vorsehen einer Benetzungsoberfläche 2 erreicht, dass flache Flüssigkeitstropfen 19 entstehen, die die Geometrie des Prallschirms 20 wenig verändern und den Durchtrittsbereich für das Aerosol nur wenig verkleinern.

Die Erfindung ist anhand der Figuren 1, 4 und 5 in Verbindung mit einer Zweistoffdüse beschrieben worden. Sie ist aber nicht auf Vernebler mit dieser Aerosolerzeugerart beschränkt.

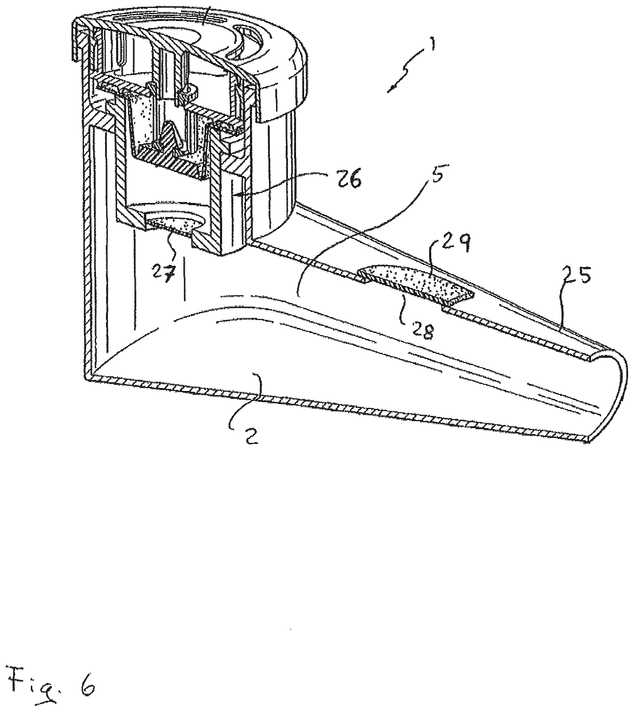

Figur 6 zeigt einen Vernebler 1, mit einem Verneblungsraum 5, einem Mundstück 25 und einem Membran-Aerosolgenerator 26. Die Schwingmembran 27 kann z. B. durch in der Figur nicht dargestellte ringförmige Piezo-Elemente in Schwingungen versetzt werden. Auf der einen Seite der Schwingmembran 27, in Fig. 6 oben, befindet sich im Betrieb des Verneblers 1 eine Flüssigkeit, die durch Öffnungen in der Schwingmembran 27 hindurch transportiert und auf der anderen Seite der Schwingmembran 27, in Fig. 6 unten, als Aerosol in den Verneblungsraum 5 abgegeben wird.

Der Verneblungsraum 5 ist mit einer Benetzungsoberfläche 2 versehen.

Der Patient kann an dem Mundstück 25, das im Verneblungsraum 5 vorhandene Aerosol abatmen. Um den Patienten nicht nach dem Einatmen des Aerosols zum Absetzen des Therapiegeräts zu veranlassen, besitzt das Mundstück 25 eine Ausatemöffnung 28, die von einem elastischen Ventilelement 29 verschlossen ist. Atmet der Patient in das Mundstück 25 und damit in den Verneblungsraum 5 hinein aus, öffnet sich das elastische Ventilelement 29, so dass die Ausatemluft aus dem Inneren des Verneblers 1 austreten kann. Beim Einatmen strömt Umgebungsluft durch den Membran-Aerosolgenerator 26 hindurch.

### Bezugszeichenliste

- 1: Vernebler
- 2: Benetzungsoberfläche
- 3: Zuluftkamin
- 4: Begrenzungswand
- 5: Verneblungsraum
- 6: Aerosolerzeuger
- 7: Zweistoffdüse,
- 8: Gaskanal
- 9: Flüssigkeitskanäle
- 10: Medikamentenreservoir
- 11: Zerstäubungsregion
- 12: Mündung
- 13: Luftstromsteuer
- 14: Einlassöffnung
- 15: Außenseite
- 16: Auslass
- 17: dicke Flüssigkeitstropfen
- 18: Durchlass
- 19: flache Flüssigkeitstropfen
- 20: Prallschirm
- 21: Prallabschnitt
- 22: Leitabschnitt
- 23: Stirnseite
- 24: Anschluss
- 25: Mundstück
- 26: Membran-Aerosolgenerator
- 27: Schwingmembran
- 28: Ausatemöffnung
- 29: Ventilelement

## Patentansprüche

1. Vernebler (1) mit einem Aerosolerzeuger (6),
einem Verneblungsraum (5),
einem Reservoir zur Bevorratung von zu vernebelnder Flüssigkeit, und
einem Auslass (16),
wobei das Reservoir eingerichtet ist, die Flüssigkeit an den Aerosolerzeuger (6) abzugeben,
wobei der Aerosolerzeuger (6) eingerichtet ist, Flüssigkeitstropfen zu erzeugen und mit Gas zu vermischen und somit Aerosol zu erzeugen und Aerosol in den Verneblungsraum (5) abzugeben, und der Auslass (16) eingerichtet ist, eine Entnahme des Aerosols aus dem Verneblungsraum (5) zu ermöglichen, wobei
in dem Verneblungsraum (5) eine Benetzungsoberfläche (2) vorgesehen ist, die eingerichtet ist, von der Flüssigkeit benetzt zu werden, und derart eingerichtet ist, dass sich Flüssigkeitstropfen, die an die Benetzungsoberfläche (2) gelangen, an die Benetzungsoberfläche (2) anlegen, sodass ein Flüssigkeitsfilm ausgebildet wird, wobei
die Benetzungsoberfläche an einem Kunststoffbauteil vorgesehen ist, **dadurch gekennzeichnet, dass** der Verneblungsraum ferner
eine Filterfläche zum Herausfiltern von Flüssigkeitstropfen aufweist, wobei größere Flüssigkeitstropfen dagegen prallen und kleinere Flüssigkeitstropfen daran vorbei geleitet werden, und die Filterfläche die Benetzungsoberfläche (2) aufweist und in einem Bereich angeordnet ist, der dafür vorgesehen ist, dass das Aerosol eine Richtungsänderung erfährt.

2. Vernebler (1) nach Anspruch 1, **dadurch gekennzeichnet, dass** der Verneblungsraum ferner eine Prallfläche (20) aufweist, und die Prallfläche (20) die Benetzungsoberfläche (2) aufweist.

3. Vernebler (1) nach Anspruch 1 oder Anspruch 2, **dadurch gekennzeichnet, dass** der Aerosolerzeuger (6) eine Düse (7) aufweist.

4. Vernebler (1) nach einem der vorhergehenden Ansprüche, **dadurch gekennzeichnet, dass** der Verneblungsraum (5) eine Aerosolführung aufweist, die den Aerosolerzeuger (6) mit dem Auslass (16) verbindet, und die Aerosolführung die Benetzungsoberfläche (2) aufweist.

5. Vernebler (1) nach Anspruch 4, **dadurch gekennzeichnet, dass** eine Fläche der Aerosolführung die Benetzungsoberfläche (2) aufweist.

6. Vernebler (1) nach Anspruch 4 oder Anspruch 5, **dadurch gekennzeichnet, dass** eine Kante der Aerosolführung die Benetzungsoberfläche (2) aufweist.

7. Vernebler (1) nach einem der vorhergehenden Ansprüche, **dadurch gekennzeichnet, dass** die Aerosolführung einen Durchlass (18) aufweist und der Durchlass (18) die Benetzungsoberfläche (2) aufweist.

8. Vernebler (1) nach einem der vorhergehenden Ansprüche, **dadurch gekennzeichnet, dass** der Verneblungsraum (5) eine Begrenzungswand (4) aufweist, die die Benetzungsoberfläche (2) aufweist.

9. Vernebler (1) nach einem der vorhergehenden Ansprüche, **dadurch gekennzeichnet, dass** das Reservoir (10) die Benetzungsoberfläche (2) aufweist.

10. Verfahren zur Herstellung eines Verneblers (1) mit einer Benetzungsoberfläche (2), umfassend die Schritte:
- Herstellen eines Verneblers (1) mit einem Aerosolerzeuger (6), einem Verneblungsraum (5), einem Reservoir zur Bevorratung von zu vernebelnder Flüssigkeit, und einem Auslass (16), wobei das Reservoir eingerichtet ist, die Flüssigkeit an den Aerosolerzeuger (6) abzugeben, wobei der Aerosolerzeuger (6) eingerichtet ist, Flüssigkeitstropfen zu erzeugen und mit Gas zu vermischen und somit Aerosol zu erzeugen und Aerosol in den Verneblungsraum (5) abzugeben, und der Auslass (16) eingerichtet ist, eine Entnahme des Aerosols aus dem Verneblungsraum (5) zu ermöglichen;
und
- Vorsehen einer Benetzungsoberfläche (2) in dem Verneblungsraum (5), die eingerichtet ist, von der Flüssigkeit benetzt zu werden, und derart eingerichtet ist, dass sich Flüssigkeitstropfen, die an die Benetzungsoberfläche (2) gelangen, an die Benetzungsoberfläche (2) anlegen, sodass ein Flüssigkeitsfilm ausgebildet wird, **dadurch gekennzeichnet, dass** der Verneblungsraum ferner
eine Filterfläche zum Herausfiltern von Flüssigkeitstropfen aufweist, wobei größere Flüssigkeitstropfen dagegen prallen und kleinere Flüssigkeitstropfen daran vorbei geleitet werden, und die Filterfläche die Benetzungsoberfläche (2) aufweist und in einem Bereich angeordnet ist, der dafür vorgesehen ist, dass das Aerosol eine Richtungsänderung erfährt.

11. Verfahren nach Anspruch 10, **dadurch gekennzeichnet, dass** der Verneblungsraum ferner eine Prallfläche (20) aufweist, und die Prallfläche (20) die Benetzungsoberfläche (2) aufweist.

## Claims

1. Nebuliser (1) with an aerosol generator (6),
a nebulising chamber (5),
a reservoir for storing liquid to be nebulised, and
an outlet (16),
wherein the reservoir is configured to dispense the liquid to the aerosol generator (6),
wherein the aerosol generator (6) is configured to generate liquid droplets and mix these with gas and thus generate aerosol and dispense aerosol into the nebulising chamber (5), and the outlet (16) is configured to allow the aerosol to be drawn from the nebulising chamber (5), wherein
a wetting surface (2) is provided in the nebulising chamber (5) which is designed to be wetted by the liquid and is configured in such a way that liquid droplets which reach the wetting surface (2) attach themselves to the wetting surface (2) so that a liquid film is formed, wherein
the wetting surface is provided on a plastics component, **characterised in that** the nebulising chamber further has a filter surface for filtering out liquid droplets, wherein larger liquid droplets strike against this and smaller liquid droplets are directed past it, and the filter surface has the wetting surface (2) and is arranged in an area intended for the aerosol to change direction.

2. Nebuliser (1) according to claim 1, **characterised in that** the nebulising chamber further has a baffle surface (20) and the baffle surface (20) has the wetting surface (2) .

3. Nebuliser (1) according to claim l or claim 2, **characterised in that** the aerosol generator (6) has a nozzle (7).

4. Nebuliser (1) according to one of the preceding claims, **characterised in that** the nebulising chamber (5) has an aerosol guide connecting the aerosol generator (6) to the outlet (16) and the aerosol guide has the wetting surface (2).

5. Nebuliser (1) according to claim 4, **characterised in that** a surface of the aerosol guide has the wetting surface (2).

6. Nebuliser (1) according to claim 4 or claim 5, **characterised in that** an edge of the aerosol guide has the wetting surface (2).

7. Nebuliser (1) according to one of the preceding claims, **characterised in that** the aerosol guide has a passage (18) and the passage (18) has the wetting surface (2) .

8. Nebuliser (1) according to one of the preceding claims, **characterised in that** the nebulising chamber (5) has a boundary wall (4) which has the wetting surface (2).

9. Nebuliser (1) according to one of the preceding claims, **characterised in that** the reservoir (10) has the wetting surface (2).

10. Method for producing a nebuliser (1) with a wetting surface (2), comprising the steps:
- producing a nebuliser (1) with an aerosol generator (6), a nebulising chamber (5), a reservoir for storing liquid to be nebulised and an outlet (16), wherein the reservoir is configured to dispense the liquid to the aerosol generator (6), wherein the aerosol generator (6) is configured to produce liquid droplets and mix these with gas and thus to generate aerosol and dispense aerosol into the nebulising chamber (5), and the outlet (16) is configured to allow the aerosol to be drawn from the nebulising chamber (5);
and
- providing a wetting surface (2) in the nebulising chamber (5) which is configured to be wetted by the liquid and is configured in such a way that liquid droplets reaching the wetting surface (2) attach themselves to the wetting surface (2) so that a film of liquid is formed, **characterised in that** the nebulising chamber further
has a filter surface for filtering out liquid droplets, wherein larger liquid droplets strike against it and smaller liquid droplets are directed past it, and the filter surface has the wetting surface (2) and is arranged in an area intended for the aerosol to change direction.

11. Method according to claim 10, **characterised in that** the nebulising chamber further has a baffle surface (20) and the baffle surface (20) has the wetting surface (2) .

## Revendications

1. Nébuliseur (1) avec un générateur d'aérosol (6),
un espace de nébulisation (5),
un réservoir pour le stockage de liquide à nébuliser, et
une sortie (16),
dans lequel le réservoir est conçu de façon à délivrer le liquide au générateur d'aérosol (6), dans lequel le générateur d'aérosol (6) est conçu de façon à générer des gouttes de liquide et à les mélanger avec du gaz et ainsi de générer un aérosol, et à délivrer l'aérosol dans l'espace de nébulisation (5), et la sortie (16) est conçue de façon à permettre un prélèvement de l'aérosol hors de l'espace de nébulisation (5), une surface d'humidification (2) étant prévue dans l'espace de nébulisation (5), laquelle est conçue de façon à être humidifiée par le liquide, et est conçue de telle manière que des gouttes de liquide qui parviennent au niveau de la surface d'humidification (2), se placent sur la surface d'humidification (2) de sorte qu'un film de liquide soit formé, la surface d'humidification étant prévue au niveau d'un composant plastique, **caractérisé en ce que** l'espace de nébulisation présente de plus une surface de filtre pour le filtrage de gouttes de liquide, dans laquelle les grosses gouttes de liquide rebondissent contre celle-ci et les petites gouttes de liquide étant dirigées au-delà de celle-ci, et la surface de filtre présentant la surface d'humidification (2) et étant agencée dans une zone qui est prévue de façon que l'aérosol subisse une modification de direction.

2. Nébuliseur (1) selon la revendication 1, **caractérisé en ce que** l'espace de nébulisation présente de plus une surface d'impact (20), et la surface d'impact (20) présente la surface d'humidification (2).

3. Nébuliseur (1) selon la revendication 1 ou la revendication 2, **caractérisé en ce que** le générateur d'aérosol (6) présente une buse (7).

4. Nébuliseur (1) selon l'une quelconque des revendications précédentes, **caractérisé en ce que** l'espace de nébulisation (5) présente un guidage d'aérosol qui relie le générateur d'aérosol (6) à la sortie (16), et le guidage d'aérosol présente la surface d'humidification (2).

5. Nébuliseur (1) selon la revendication 4, **caractérisé en ce qu'**une surface du guidage d'aérosol présente la surface d'humidification (2).

6. Nébuliseur (1) selon la revendication 4 ou la revendication 5, **caractérisé en ce qu'**une arête du guidage d'aérosol présente la surface d'humidification (2).

7. Nébuliseur (1) selon l'une quelconque des revendications précédentes, **caractérisé en ce que** le guidage d'aérosol présente un passage (18) et le passage (18) présente la surface d'humidification (2).

8. Nébuliseur (1) selon l'une quelconque des revendications précédentes, **caractérisé en ce que** l'espace de nébulisation (5) présente une paroi de limitation (4) qui présente la surface d'humidification (2).

9. Nébuliseur (1) selon l'une quelconque des revendications précédentes, **caractérisé en ce que** le réservoir (10) présente la surface d'humidification (2).

10. Procédé de fabrication d'un nébuliseur (1) avec une surface d'humidification (2), comprenant les étapes suivantes :
- le fait de fabriquer un nébuliseur (1) avec un générateur d'aérosol (6), un espace de nébulisation (5), un réservoir pour le stockage de liquide à nébuliser et une sortie (16), dans lequel le réservoir est conçu de façon à délivrer le liquide au générateur d'aérosol (6), le générateur d'aérosol (6) est conçu de façon à générer des gouttes de liquide et à les mélanger avec du gaz, et à ainsi générer un aérosol et délivrer l'aérosol dans l'espace de nébulisation (5), et la sortie (16) est conçue de façon à permettre un prélèvement de l'aérosol hors de l'espace de nébulisation (5) ;
et
- le fait de prévoir une surface d'humidification (2) dans l'espace de nébulisation (5), qui est conçue de façon à être humidifiée par le liquide, et est conçue de telle manière que des gouttes de liquide qui parviennent au niveau de la surface d'humidification (2) se placent sur la surface d'humidification (2) de sorte qu'un film de liquide soit réalisé, **caractérisé en ce que** l'espace de nébulisation présente de plus une surface de filtre pour le filtrage des gouttes de liquide, dans lequel les grosses gouttes de liquide rebondissent contre celle-ci et les petites gouttes de liquide sont dirigées au-delà de celle-ci, et la surface de filtre présente la surface d'humidification (2) et est agencée dans une zone qui est prévue de façon que l'aérosol subisse une modification de direction.

11. Procédé selon la revendication 10, **caractérisé en ce que** l'espace de nébulisation présente de plus une surface d'impact (20), et la surface d'impact (20) présente la surface d'humidification (2).
